# EUROPEAN PATENT SPECIFICATION

(11) **EP 1 004 025 B1**
(45) Date of publication and mention of the grant of the patent: **14.01.2004**
(21) Application number: 99931860.3
(22) Date of filing: 23.06.1999
(51) Int. Cl.: G01N 35/02, G01N 33/52

(54) **MULTI-LAYER TESTING COLUMN**
TESTSÄULE MIT MEHREREN LAGEN
COLONNE D'ANALYSE MULTICOUCHE

(30) Priority: 24.06.1998 US 90469 P
(43) Date of publication of application: 31.05.2000
(73) Proprietor: Chen & Chen, LLC, Brookline, MA 02446 (US)
(72) Inventor: CHEN, Shuqi, Brookline, MA 02446 (US)
(74) Representative: Horner, Martin Grenville
(86) International application number: PCT/US1999/014118
(87) International publication number: WO 1999/067647

(56) References cited:
- EP-A- 0 139 373
- EP-A- 0 312 394
- WO-A-94/20831
- US-A- 5 057 438

## Description

### INTRODUCTION

The present invention is directed to a testing column, and, more particularly, to a multi-layered testing column capable of identifying and quantifying a number of analytes of a fluid sample in a single test.

### BACKGROUND

Assays are commonly used to test fluid samples, such as blood, in order to determine the presence and/or quantity of an analyte in the fluid sample. To test a sample of blood for the existence of an analyte, e.g., an antigen, an antibody, a ligand or ligand receptor, current testing uses microtiter, or microplate technology, where a single test is performed on a fluid sample at each of numerous separate sites. A device often used in microplate testing is a so-called ninety-six well microplate, an apparatus having ninety six recesses, or wells, into each of which a quantity of a particular anti-analyte, e.g., an antibody or an antigen or the like, is placed and is attached to the well. A quantity of sample is then placed in each well. Specific analytes which may be present in the sample (e.g., antibodies, antigens, ligands and receptors) bind to the anti-analyte in each well, revealing the existence of the analyte when a reagent conjugated to a detectable label is added to the well. The binding efficiency of the sample to the anti-analyte in such a device may be relatively low, and operation of these devices is costly. Such a test is "one-to-one" based, that is, one test is required for the detection of each specific analyte. The one-to-one test platform is also widely used to test for biological activity of compounds, e.g., searching for potential new drugs. Since there are hundreds of thousands of chemical compounds that need to be screened, performance of one-to-one tests for drug discovery takes a tremendously long time.

EP-A-0139373 relates to a microassay rod adapted for use in screening of biologic fluid and other sample fluids or gasses for the presence of substances which are capable of undergoing specific binding reactions.

EP-A-0312394 relates to membrane-supported immunoassays employing a reuseable syringe or vacuum manifold to pass samples by means of a pressure gradient through a membrane containing an affinity for analyte.

US-A-5057438 relates to the determination of a plurality of species of antibodies or antigens attained by a method which comprises forming a plurality of different kinds of reaction membranes each having a different species of antibody or antigen on an electrophoretic carrier and superposing these reaction membranes.

It is an object of the present invention to provide a multi-layered testing column which reduces or wholly overcomes some or all of the aforesaid difficulties inherent in prior known devices. Particular objects and advantages of the invention will be apparent to those skilled in the art, that is, those who are knowledgeable or experienced in this field of technology, in view of the following disclosure of the invention and detailed description of certain preferred embodiments.

### SUMMARY

The principles of the invention may be used to advantage to provide a multi-layer testing column allowing multiple tests to be performed on a single fluid sample substantially simultaneously, providing improved efficiency and reduced costs.

According to a first aspect of the present invention there is provided a multi-layer column comprising, in combination:
a chamber having a longitudinal axis with a first end having a first aperture; and
a plurality of vertically stacked, substantially planar filter-like microporous membrane layers stacked within the chamber, including at least a plurality of solid-phase substrates each carrying a different anti-analyte and each membrane layer having a plurality of pores which allows sample fluid to flow through each membrane layer, and permits the sample fluid to flow through the plurality of vertically stacked membrane layers, the plane of each of the membrane layers being substantially perpendicular to the longitudinal axis of the chamber.

According to a second aspect of the present invention there is provided a fluid-testing apparatus comprising, in combination:
a housing;
mounting means within the housing to receive an elongate chamber;
an elongate chamber mounted in the mounting means, having a first aperture at a first end thereof and a plurality of vertically stacked, substantially planar filter-like microporous membrane layers stacked within the chamber, including at least a plurality of solid-phase substrates each carrying a different anti-analyte and each membrane layer having a plurality of pores which allows sample fluid to flow through each membrane layer, and permits the sample fluid to flow through the plurality of vertically stacked membrane layers, the plane of each of the membrane layers being substantially perpendicular to the longitudinal axis of the chamber;
a reagent reservoir and fluid delivery means within the housing to feed reagent from the reagent reservoir to the first aperture of the elongate chamber;
a wash buffer reservoir and second fluid delivery means within the housing to feed wash buffer from the wash buffer reservoir to the first aperture of the elongate chamber; and
a sensor within the housing to receive a signal from the elongate chamber and to generate a corresponding electrical signal.

From the foregoing disclosure, it will be readily apparent to those skilled in the art, that is, those who are knowledgeable or experienced in this area of technology, that the present invention provides a significant technological advance. Preferred embodiments of the multi-layer testing column of the present invention can provide enhanced binding efficiency, increased surface area for capture of analytes of a sample, and reduced costs. These and additional features and advantages of the invention disclosed here will be further understood from the following detailed disclosure of certain preferred embodiments.

### BRIEF DESCRIPTION OF THE DRAWINGS

Certain preferred embodiments are described in detail below with reference to the appended drawings wherein:
Fig. 1 is a schematic perspective view of a multi-layer testing apparatus according to the present invention;
Fig. 2 is a schematic elevation view, shown partially in section. of a multi-layer column according to the present invention;
Fig. 3 is a schematic perspective view of a column of membrane layers for insertion into the multi-layer filter chamber of Fig. 2 and formed from a plurality of stacked sheets of coated substrate;
Fig. 4 is a schematic section view of portion of the column of membrane layers of Fig. 3;
Fig. 5 is a schematic representation of the multi-layer testing apparatus of Fig. 1;
Fig. 6 is a schematic perspective view of the housing of the multi-layer column of Fig. 2. shown inserted into a waste bin;
Fig. 7 is a schematic perspective view. shown partially in phantom. of an alternative embodiment of the housing and membrane layers of the multi-layer column of Fig. 2; which is not part of the present invention;
Fig. 8 is a schematic perspective view, shown partially in section, of another alternative embodiment of the housing and membrane layers of the multi-layer column of Fig. 2;
Fig. 9 is a schematic section view of an alternative embodiment of the membrane layers of the multi-layer column of Fig. 2; and
Fig. 10 is a schematic elevation view of a conveyor carrying multi-layer columns to the multi-layer testing apparatus of Fig. 5
The figures referred to above are not drawn necessarily to scale and should be understood to present a representation of the invention, illustrative of the principles involved. Some features of the multi-layer testing apparatus depicted in the drawings have been enlarged or distorted relative to others to facilitate explanation and understanding. The same reference numbers arc used in the drawings for similar or identical components and features shown in various alternative embodiments. Multi-layer testing apparatuses as disclosed herein, will have configurations and components determined, in part, by the intended application and environment in which they are used.

### DETAILED DESCRIPTION OF CERTAIN PREFERRED EMBODIMENTS

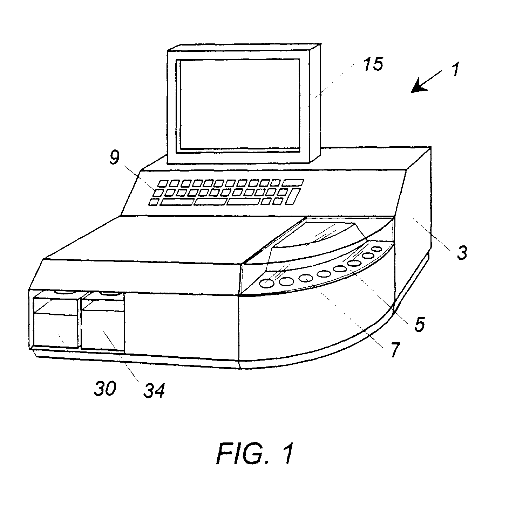

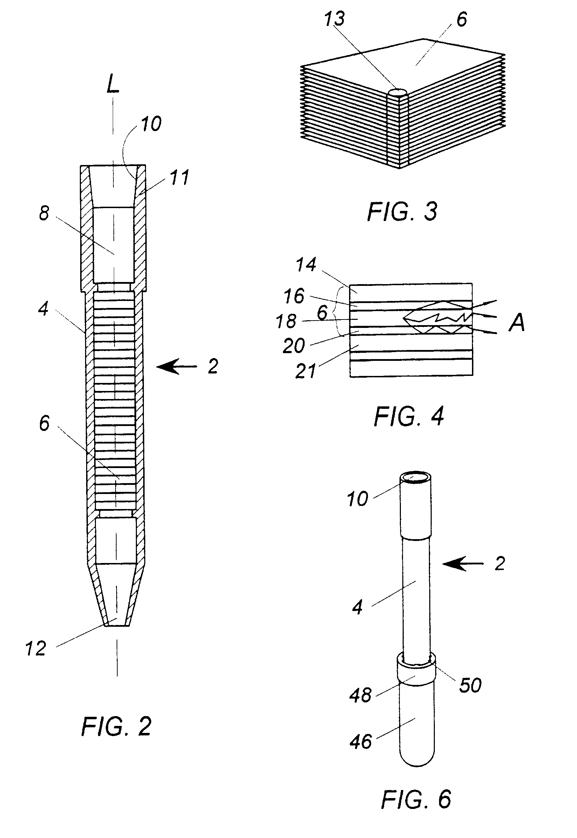

Referring to Fig. 1, a testing machine according to the present invention is shown generally by the reference numeral 1. Testing machine 1 comprises housing 3 having a mounting means and transport means such as rotatable loading wheel 5 having recesses 7 to receive elongate columns 2 (seen in Fig. 2 and described in greater detail below). Loading wheel 5 allows a plurality of columns 2 to be loaded into testing machine 2 and transported to stations within testing machine 1 for testing of a fluid sample. Other suitable mounting means and transport means may include. for example, a conveyor track or belt or a movable gripping arm which can move an elongate column between stations in testing machine 1. Other suitable mounting and transport means will become readily apparent to those skilled in the art, given the benefit of this disclosure. The elongate columns may be, for example, test tube shaped devices containing within them multi-layer membranes as described further below. Testing machine 1 has a control panel 9 to receive information, such as information read from bar code labels or keyed data, and a monitor 15 to display operating information, such as the results of testing.

A multi layer testing column 2, seen in Fig. 2, is contained within housing 3. Testing column 2 comprises elongate, cylindrical chamber or housing 4 having a longitudinal axis L and containing a plurality of membrane layers 6. A reservoir 8 is positioned above the membrane layers 6. Aperture 10 is formed at the upper end of housing 4. Aperture 12 is formed at the lower end of housing 4. A pipette adapter 11 is connected to the upper end of housing 4.

As seen in Fig. 3, membrane layers 6 are preferably formed by stacking a plurality of sheets of filter-like microporous membrane or fiber matrix membranes. Stacked membranes, as used herein, refer to a plurality of membranes placed in overlapping fashion. It is to be appreciated that the membranes may be offset with respect to one another, and that the membrane layers may be spaced from one another. for example, by spacer layers. A filter-like microporous membrane, as used herein. refers to a membrane which provides solid support for an anti-analyte, and has a plurality of pores which allow sample fluid to flow through the membrane and provide increased surface area for attachment of the anti-analyte as well as an increased number of sites for binding of analyte to anti-analyte. A fiber matrix membrane, as used herein, refers to a membrane formed of a mat of fibers. preferably a fabric, which provides solid support for an anti-analyte, and has a plurality of pores which allow sample fluid to flow through the membrane and provide increased surface area for attachment of the anti-analyte as well as an increased number of sites for binding of analyte to anti-analyte. The membrane layers are preferably transparent to light and in general should be stackable in a chamber. Suitable materials for membrane layer 6 include cellulose. nitrocellulose, acrylic copolymer, polyethersulphone, polyethylene. polyvinylidene fluoride, polymer, nylon, plastic, and glass.

Analyte, as used herein, refers to a substance present in a fluid sample and is sometimes referred to herein as a target analyte or target material. In accordance with certain preferred embodiments, exemplary analytes include antibodies, antigens, ligands, ligand receptors, other proteins, and nucleic acids. Other suitable analytes will become readily apparent to those skilled in the art, given the benefit of this disclosure. Antianalyte, as used herein, refers to a substance which reacts specifically with an analyte present in a fluid sample or to which an analyte in a fluid sample binds. Exemplary anti-analytes include antibodies, antigens, ligands, ligand receptors, aptamers, nucleic acids capable of hybridizing to an analyte nucleic acid, enzyme-linked immunosorbent analyte proteins or fragments or proteins capable of forming a complex with an analyte protein or protein fragment. and chemical compounds capable of having biological activity with a target analyte. Other suitable anti-analytes will become readily apparent to those skilled in the art, given the benefit of this disclosure. Corresponding analyte and anti-analyte pairs include, for example:
antibody, for which a typical anti-analyte would be an antigen which specifically binds with the target antibody;
other proteins or fragments of proteins capable of forming a complex with a target protein or a target protein fragment;
nucleic acid able to hybridize to a target nucleic acid;
chemical compounds, which may potentially be able to act on a biological target linked to a particular disease: and
indicators for sample constituents or sample conditions such as pH.

It will be understood by those skilled in the art that antibodies, or fragments of antibodies. can bind to an antigen or fragment of an antigen, e.g., a target protein. lipid. amino acid. phosphate group, carbohydrate, etc. Additionally, it will be understood that typically a target nucleic acid, for example, from a blood cell, would first he extracted from the blood cell by any of several known techniques. It is possible. for example, to sonicate a sample or subject it to a detergent or organic extraction. The resulting lysate would then be injected into a multi-layer testing column. Such lysate may or may not still contain cell wall material and other cell debris. As discussed below, preferred embodiments of the multi-layer testing column of the invention employ filter layers to filter out such debris from the fluid sample which has been injected or otherwise fed into the multi-layer testing column.

At least some sheets are coated with an anti-analyte to serve as a solid-phase substrate. Attachment of the anti-analyte to the substrate may be carried out by any conventional procedure, such as, for example, absorption or covalent bonding. A membrane surface can be chemically treated and certain anti-analytes may be chemically linked to the substrate. Chemical compounds can be directly synthesized on a membrane. These procedures are well known in the art, and no further details in these respects are deemed necessary for a complete understanding of the invention. In certain preferred embodiments, the anti-analyte is biologically active.

The anti-analyte attaches to membrane layer 6 and provide sites to which analyte of a fluid sample binds. The anti-analyte coating membrane layers 6 preferably provides specific binding sites. Specific binding sites, as used herein, refer to sites to which a specific analyte or class of analytes bind. The membrane layer 6 is then treated with a blocking substance or reagent to substantially block the non-specific binding sites on the membrane layer. Thus, the anti-analyte remains available for binding with a target analyte. but the non-specific binding sites are substantially blocked. The blocking reagent may be, e.g., bovine serum albumen (BSA).

The stack of membrane layers 6 is then cut into a plurality of columns 13 sized to fit within housing 4. In certain preferred embodiments, as seen in Fig. 4. each membrane layer 6 preferably comprises a plurality of sub-layers, specifically, a shielding layer formed of a light absorption layer 14 and a first light reflective layer 16 below absorption layer 14; a capture layer 18 to which the coated anti-analyte attaches: and a second shielding layer formed of a second reflective layer 20 below capture layer 18 and a second absorption layer 21 below second reflective layer 20. Each shielding layer may, in certain preferred embodiments, act as a shielding layer for capture layers both above and below the shielding layer. Capture layer 18 is substantially transparent to at least certain wavelengths of light. Light which is emitted by capture layer 18 (described in greater detail below) is substantially reflected out of membrane layer 6 by reflective layers 16, 20 as shown by arrows A. Absorption layer 14 absorbs light emitted by capture layer 18. Thus, reflective layers 16, 20 and absorption layer 14 substantially prevent light emitted from a capture layer 18 from entering capture layers 18 of adjacent membrane layers 6. The uppermost and lowermost membrane layers 6 in housing 4 are preferably filter layers which substantially prevent passage of large particles. e.g. blood cells, to other membrane layers 6 in housing 4. They do not. however. filter or block the flow of other, smaller sample analytes, e.g., antibodies. etc., which are passed to the lower membrane layers for binding.

Certain membrane layers 6 may be treated only with a blocking substance or reagent to substantially block non-specific binding sites and serve as negative control layers. Negative control layers, under normal operating conditions, produce substantially no emitted light. If substantial light is emitted from a negative control layer, it is an indication that a problem exists within column 2. Other membrane layers 6 may be coated specifically to act as positive control layers. A positive control layer, under normal operating conditions, produces an emitted light. Thus, if conditions are other than normal, there will be substantially no emitted light from the positive control layer, indicating that a problem exists within column 2. The control layers can be used, for example, as a reference to quantify the amount of the analyte in the sample by comparing the magnitude of the light or signal generated by the control layer to the magnitude of the light or signal generated by the capture layer. This relationship works when the magnitude of the signal is established for a known quantity of analyte. The control layers can also be used to identify faulty label and/or reagent, to indicate contamination of column 2, or serve as positional markers between membrane layers 6. For instance, in a column 2 having hundreds of stacked membrane layers 6, a control layer could be positioned between every ten membrane layers 6. reducing the chance of accumulated error which could occur in incorrectly identifying a particular membrane layer within a long string of adjacent membrane layers.

Housing 4 is preferably filled with a buffer solution to stabilize the coated membrane layers. Typically, the buffer solution is removed from housing 4 prior to use. Suitable buffer solutions include, e.g.. phosphate buffers supplemented with BSA.

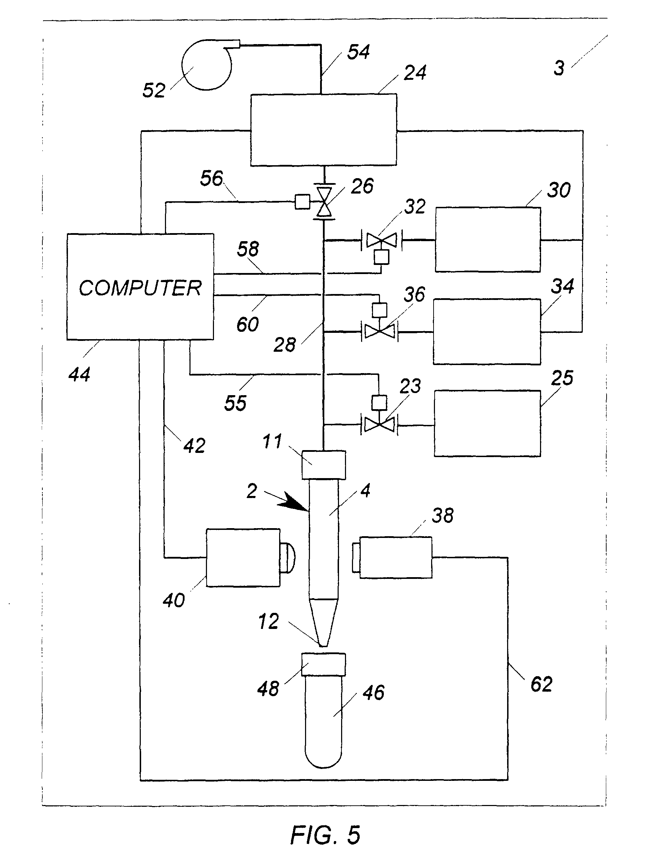

Turning now to Fig. 5. components housed within housing 3 of testing machine 1 are shown. A sample reservoir 25 is connected by control valve 23 and conduit 28 into column 2. Sample reservoir 25 may be, in certain preferred embodiments. a fluid sample container such as a test tube, typically used for collecting a fluid sample, e.g., patient's blood. Such a fluid sample container or test tube may be housed in a mounting means such as the recesses 7 of loading wheel 5 described above. Fluid sample can be removed from a test tube and inserted in a multi-layer column 2 by known fluid delivery means. Exemplary fluid delivery means include, for example, a vacuum based pipette tip or sampling needle which is connected to a pipette adapter and inserted into a test tube to draw fluid from the test tube; a movable arm to move the needle to a multi-layer column 2; and a pressure source to force fluid sample from the needle into the first aperture 10 of the housing 4 of multi-layer column 2. Other suitable fluid delivery means will become readily apparent to those skilled in the art given the benefit of this disclosure.

In certain preferred embodiments, a loading wheel 5 may be provided for handling multi-layer columns 2 and a separate loading wheel 5 may be provided for handling test tubes or other containers of fluid sample. Thus. a single, or multiple loading wheels 5 may be used to transport a combination of multi-layer columns 2 and fluid sample containers between different stations within testing machine 1. Exemplary stations within testing machine 1 include a fluid handling station where fluid sample is taken from a fluid sample container and transferred to a multi-layer column, a reagent station where reagent is added to the multi-layer column, a washing station where wash buffer is added to the multi-layer column to remove unbound analyte and/or other particles, and a sensing station where light or another signal from multi-layer column 2 is received. In other preferred embodiments. multiple processing steps can be performed at a single station.

An air reservoir 24 is connected by control valve 26 and conduit 28 to column 2. Wash buffer reservoir 30 is connected by control valve 32 and conduit 28 to column 2. Reagent reservoir 34 is connected by control valve 36 and conduit 28 to column 2. A sensor is used to receive a signal from column 2 and generate a corresponding electrical signal. In the illustrated embodiment, the sensor comprises a light source 38 and a light sensor 40. An excitation energy source is provided in certain preferred embodiments to direct excitation energy toward column 2 to generate a signal from column 2. In the illustrated embodiment, the energy source is light source 38. The excitation energy source may, in certain preferred embodiments, apply a voltage differential across the elongate column via a pair of electrodes, not shown. Other suitable excitation energy sources will become readily apparent to those skilled in the art, given the benefit of this disclosure.

Light source 38 is positioned adjacent column 2 with light sensor 40 positioned adjacent column 2, opposite light source 38. Light sensor 40 is connected by cable 42 to computer 44 located in housing 3. Reservoirs 25, 24, 30, 34 serve to provide containment for a suitable volume of sample, air, wash buffer or reagent. In certain preferred embodiments, the reservoirs contain enough sample. air. wash buffer or reagent for a single test, and, more preferably, are sized to contain enough for multiple tests. The reservoirs provide a supply of at least a portion of their contents which can be fed through a conduit, which is optionally regulated by a control valve, to column 2. Suitable reservoirs will become readily apparent to those skilled in the art. given the benefit of this disclosure.

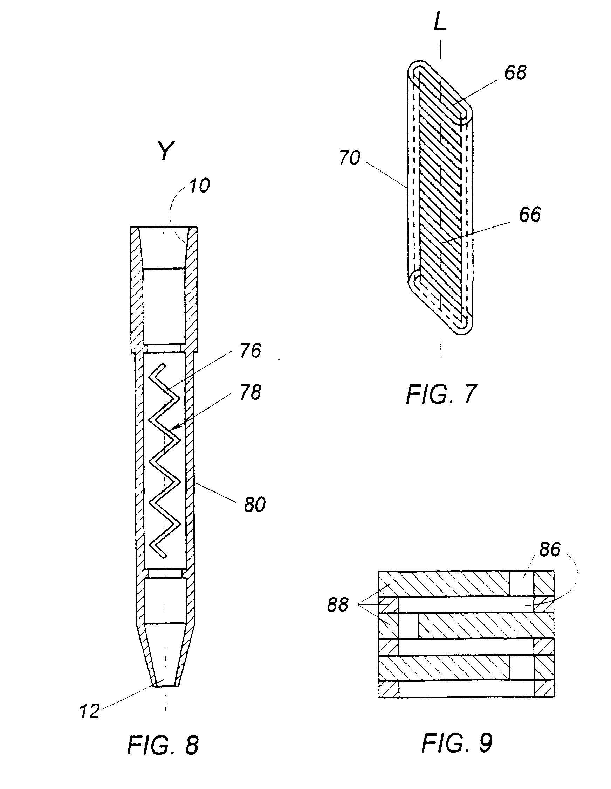

Waste reservoir or bin 46 is positioned below column 2 to receive outflow from aperture 12. In certain preferred embodiments, as seen in Fig. 6. waste bin 46 is directly connected to the lower end of housing 4 with collar 48. Channels 50 are formed in collar 48 to allow the passage of air into and out of column 2.

The operation of testing machine I will now be described with respect to Fig. 5. As noted above. a sample. for example, a blood sample. is introduced into column 2 from sample reservoir 25. Air is introduced into column 2 from air reservoir 24 through conduit 28. Pump 52 is connected to air reservoir 24 by conduit 54 to vary the air pressure in reservoir 24. The air pressure in column 2 is varied alternately from a positive value to a vacuum, causing the sample to flow up and down through membrane layers 6. Each capture layer 18 of the membrane layers 6, having one anti-analyte coated thereon, binds a specific analyte, e.g., a specific antigen, antibody, ligand or receptor, etc. present in the sample. The flow rate of the sample through column 2 is controlled in order to enhance its binding efficiency. Binding is also increased due to the fact that the probability of analytes in the sample encountering binding sites on capture layer 18 is increased with multiple passes of the sample through column 2.

Wash buffer from reservoir 30 may be then introduced into column 2 to remove or wash unbound analytes from column 2 out through aperture 12 into waste bin 46. Reagent from reservoir 34 is then introduced into column 2. The reagent (e.g.. a second antibody) is conjugated to a detectable label which provides identification of the particular analyte present in the sample. Suitable labels include, for example, enzyme labels, and luminescent labels such as chemilluminescent labels or fluorescent labels. The labeled reagent is bound to the captured analytes by passing the labeled reagent through column 2 in a similar manner described above with respect to the sample. The flow of reagent through the chamber is, therefore, also controlled to enhance its binding efficiency. Wash buffer may be introduced again to wash out any unbound labeled reagent.

In certain preferred embodiments, the reagent has a radioactive label. In such an embodiment a sensor is employed to detect the radiation emanating from particular membrane layers 6. It is apparent that any such sensor should be sensitive enough to distinguish between the radiation emanating from different membrane layers 6. In other preferred embodiments, the reagent has a fluorescent label, in which case another light source is required for the labeled reagent to emit light. In that case, light source 38 is used to project light, e.g., a laser light of a specific wavelength, into column 2 to excite the bound, labeled reagent to produce an emitted light. In either case, capture layer 18 emits light having a specific wavelength. Light sensor 40 scans each membrane layer 6 in column 2, detecting the location of each of the captured layers based on the wavelength of its emitted light, in bar code reader fashion. A signal is then transmitted from light sensor 40 through cable 42 to computer 44 for analysis. With the labeled reagent bound to the captured analyte, light sensor 40 and computer 44 are able to detect the presence and quantity of different captured analytes on different membrane layers 6.

In other preferred embodiments, the reagent may have an electrochemiluminescent label. in which case a voltage is applied to column 2, resulting in emitted light of a specific wavelength from the bound, labeled reagent present on capture layers 18 of membrane layers 6.

In other preferred embodiments, the label may be formed of two portions, the first portion being attached to the anti-analyte on capture layer 18, the second portion being conjugated to the reagent. The label will only emit light when the first and second portions are sufficiently close to one another. An example of a suitable first portion is europium cryptate. An example of a suitable second portion is allophycocyanin. If a analyte in the sample has bound to capture layer 18 and the reagent has in turn bound to the sample analyte, the first and second portions will be close enough to create emitted light of a specific wavelength. As described above, the light may be emitted directly, or require another light source or applied voltage in order to create the emitted light. Accordingly, the presence of the analyte will be identified by the emitted light.

In certain preferred embodiments, the wash buffer and reagent can be added to column 2 manually rather than from reservoirs controlled by valves and/or a computer or other suitable controller. Similarly. sample can be added to column 2 in a manual basis, or under control of a computer or other suitable controller.

A multi-layer testing apparatus in accordance with the present invention can be used, for example, to analyze blood samples and to screen chemical compounds in search of biological activity for drug discovery. Tests that can be run on a blood sample using the present invention include, for example, tests screening for viruses, antigens, antibodies, proteins, enzymes, etc., in the blood, such as screening for Hepatitis B Surface Antigen (HbsAg), Hepatitis C Antibody (HCV), and HIV. Chemical compounds can be synthesized directly on a membrane. Then. multi-layer columns can be assembled and used to screen biological activity of the compounds with a target protein related to a disease present in a test fluid sample. By having multiple distinct layers, multiple tests can be done on a single fluid sample simultaneously. increasing productivity, throughput and reducing costs. The number of layers of membrane 6 may be in the tens, hundreds. thousands or more.

Use of a microporous membrane layer provides a larger surface area than that of a microplate, and, therefore. the sensitivity of the testing is greatly increased since the sensitivity is proportional to the capture surface. Sensitivity can also be increased through the use of labels such as chemiluminescent labels.

The present invention is suitable for use in multiple step processes, e.g., two step processes. One example of a two step process is an enzyme-linked immunosorbent assay (ELISA). In such a process, an anti-analyte is attached to a capture layer of a multi-layer column such as described above in connection with Figs. 3, 4. The column is then exposed to a sample, e.g., a patient's blood, such that analyte within the sample can bind to the anti-analyte. The column is then washed with a buffer to remove unbound analyte, using, e.g., in the embodiment described above, buffer solution from wash buffer reservoir 30. The column is then exposed to a reagent, e.g., from reagent reservoir 34, carrying the same or different anti-analyte as that coated on the capture layer, which specifically binds with the analyte. In a preferred embodiment, the reagent is labeled with a detectable moiety. Detectable moieties, include, for example, enzyme labels, radioactive labels, fluorescent labels, and chemilluminescent labels which are bound to the reagent. Optionally, the column may be washed again, using, for example, a buffer solution such as wash buffer from wash buffer reservoir 30. A sensor is then used to detect the presence and/or quantity of analyte in the sample in the manner described above.

In certain preferred embodiments, a programmable control system, e.g., a general purpose computer with suitable control software or a dedicated computer module within housing 3 may be used to automatically control testing machine 1 and its components. For example, computer 44 may be used to control operation of valves 23, 26, 32, and 36 through cables 55, 56, 58, and 60, respectively. Computer 44 may also be used to control operation of light source 38 through cable 62. It will be within the ability of those skilled in the art to provide suitable control software and hardware for controlling testing machine 1.

In certain preferred embodiments. a centrifuge device can be included within housing 3 to provide force to generate flow of sample through multi-layer membranes of a multi-layer column.

In certain preferred embodiments, testing machine 1 has a temperature controlling device. A temperature controlling means heats and/or cools a multi-layer column. It is to be appreciated that the temperature of the multi-layer column may be controlled directly, such as with a temperature sensor detecting the temperature of the multi-layer column and maintaining a desired setpoint temperature. Alternatively, the temperature of the multi-layer column could be controlled indirectly by sensing and controlling the temperature in an area housing the multi-layer column. Temperature controlling means may include a heating element and may also include a cooling device. Other suitable temperature controlling means will become readily apparent to those skilled in the art. given the benefit of this disclosure.

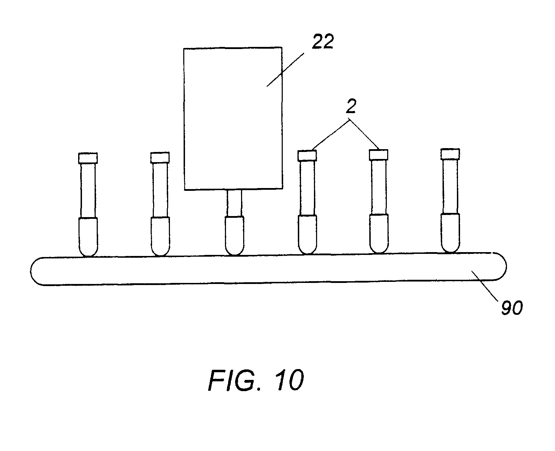

Optionally, as seen in Fig. 10, a series of columns 2 or fluid sample containers can be carried by a conveyor track or belt 90 (represented schematically here) or the like between different stations within testing machine to test separate samples of the same or different fluids. Other means for transporting multi-layer columns 2 and fluid sample containers include, for example, movable arms which can grip and hold columns 2 and the fluid sample containers.

Another embodiment, which is not part of the present invention, is shown in Fig. 7. Membrane layers 66 are formed on a single planar substrate 68 by coating different segments of the planar substrate 68 with different anti-analytes in a known manner. Planar substrate 68 is placed in housing 70 such that the plane of substrate 68 is substantially parallel to a longitudinal axis L of housing 70. Housing 70 is used in a testing apparatus as described above. Membrane layers 66 may be marked with a dye to differentiate one membrane layer 66 from another. In another preferred embodiment. the specific antigens or antibodies of the membrane layers may be placed directly on the inner surface of housing 70, which then serves as the substrate.

Another preferred embodiment is shown in Fig. 8, where membrane layers 76 are formed on a substrate 78. Substrate 78 is folded in accordion fashion within housing 80. Each membrane layer 76 is. therefore. at an oblique angle with respect to a longitudinal axis Y of housing 80. The multi-layer testing apparatus of Fig. 7 is used in a testing apparatus as described above. It is to be appreciated that in certain preferred embodiments, membrane layers 76 at the top and bottom of substrate 78 may act as filter layers as described above.

Another preferred embodiment is shown in Fig. 9, where a portion 86 of each membrane layer 88 is cut away, each portion 86 at least partially overlapping adjacent portions 86 such that a channel is formed through membrane layers 88 through which fluid sample may directly flow.

## Claims

1. A multi-layer column comprising, in combination:
a chamber having a longitudinal axis with a first end having a first aperture; and
a plurality of vertically stacked, substantially planar filter-like microporous membrane layers stacked within the chamber, including at least a plurality of solid-phase substrates each carrying a different anti-analyte and each membrane layer having a plurality of pores which allows sample fluid to flow through each membrane layer, and permits the sample fluid to flow through the plurality of vertically stacked membrane layers, the plane of each of the membrane layers being substantially perpendicular to the longitudinal axis of the chamber.

2. The multi-layer column according to claim 1, wherein each of the membrane layers comprises a fiber matrix membrane.

3. The multi-layer column according to claim 1, wherein the membrane layers are substantially transparent to light of at least a selected wavelength.

4. The multi-layer column according to claim 1, wherein each of a plurality of the membrane layers comprises a capture layer carrying anti-analyte and at least one light shielding layer substantially coplanar with the capture layer.

5. The multi-layer column according to claim 4, wherein at least one of the light shielding layers include a light absorption sub-layer.

6. The multi-layer column according to claim 4, wherein at least one of the light shielding layers includes a light reflection sub-layer.

7. The multi-layer column according to claim 1, wherein at least a plurality of the solid-phase substrates carry a blocking substance.

8. The multi-layer column according to claim 1, wherein substantially all surfaces within the chamber carry a blocking substance.

9. The multi-layer column according to claim 1, wherein endmost membrane layers of the plurality of membrane layers are filter layers having pores sized to substantially prevent flow of particles of a preselected size to central membrane layers of the plurality of membrane layers.

10. The multi-layer column according to claim 1, wherein the membrane layers are axially spaced from one another along the longitudinal axis.

11. The multi-layer column according to claim 1, further comprising a buffer solution contained within the chamber.

12. The multi-layer column according to claim 1, wherein the chamber has a second aperture at a second end thereof.

13. The multi-layer column according to claim 12, further comprising a waste reservoir in fluid communication with the second aperture of the chamber.

14. The multi-layer column according to claim 1, further comprising a fluid flow port to provide a fluid exit flow path from within the chamber.

15. The multi-layer column according to claim 1, wherein each of the anti-analytes is selected from the group consisting of antibodies, antigens, ligand, ligand receptors, nucleic acids capable of hybridizing to an analyte nucleic acid, enzyme-linked immunosorbent analyte, proteins or fragments of proteins capable of forming a complex with an analyte protein or protein fragment, and chemical compounds capable of having biological activity with a target analyte.

16. A fluid-testing apparatus comprising, in combination:
a housing;
mounting means within the housing to receive an elongate chamber;
an elongate chamber mounted in the mounting means, having a first aperture at a first end thereof and a plurality of vertically stacked, substantially planar filter-like microporous membrane layers stacked within the chamber, including at least a plurality of solid-phase substrates each carrying a different anti-analyte and each membrane layer having a plurality of pores which allows sample fluid to flow through each membrane layer, and permits the sample fluid to flow through the plurality of vertically stacked membrane layers, the plane of each of the membrane layers being substantially perpendicular to the longitudinal axis of the chamber;
a reagent reservoir and fluid delivery means within the housing to feed reagent from the reagent reservoir to the first aperture of the elongate chamber;
a wash buffer reservoir and second fluid delivery means within the housing to feed wash buffer from the wash buffer reservoir to the first aperture of the elongate chamber; and
a sensor within the housing to receive a signal from the elongate chamber and to generate a corresponding electrical signal.

17. The fluid testing apparatus according to claim 16, further comprising a computer responsive to the electrical signal generated by the sensor.

18. The fluid testing apparatus according to claim 16, further comprising a fluid handling system comprising a fluid sample container mounting means to mount at least one container of fluid sample, and fluid sample delivery means to feed fluid sample from the container of fluid sample to an elongate chamber mounted in the mounting means.

19. The fluid testing apparatus according to claim 18, wherein the fluid sample container mounting means simultaneously holds a plurality of containers of fluid sample and the fluid sample delivery means is adapted to sequentially feed fluid sample from each of the containers of fluid sample to a corresponding elongate chamber.

20. The fluid testing apparatus according to claim 16, further comprising an excitation energy source within the housing to direct excitation energy toward the elongate chamber.

21. The fluid testing apparatus according to claim 20, wherein the excitation energy source comprises a light source oriented to illuminate at least one of the membrane layers in the elongate column with light of a preselected wavelength, wherein at least one of the membrane layers in the elongate chamber is responsive to the preselected wavelength.

22. The fluid testing apparatus according to claim 20, wherein the excitation energy source applies a voltage differential across the elongate chamber.

23. The fluid testing apparatus according to claim 16, further comprising a variable pressure source operably connected to the first aperture.

## Patentansprüche

1. Säule mit mehreren Lagen, die in Kombination aufweist:
eine Kammer mit einer Längsachse mit einem ersten Ende, das eine erste Öffnung aufweist; und
eine Vielzahl von vertikal gestapelten, im wesentlichen ebenen filterartigen mikroporösen Membranlagen, die innerhalb der Kammer gestapelt sind, die mindestens eine Vielzahl von Trägermaterialien in fester Phase umfaßen, wobei jedes ein unterschiedliches Antianalyt trägt, und wobei jede Membranlage eine Vielzahl von Poren aufweist, die den Fluß eines Probefluids durch jede Membranlage gestatten, und was gestattet, daß das Probefluid durch die Vielzahl der vertikal gestapelten Membranlagen fließt, wobei die Ebene einer jeden der Membanlagen im wesentlichen senkrecht zur Längsachse der Kammer verläuft.

2. Säule mit mehreren Lagen nach Anspruch 1, bei der eine jede der Membranlagen eine Fasergrundmassemembran aufweist.

3. Säule mit mehreren Lagen nach Anspruch 1, bei der die Membranlagen im wesentlichen lichtdurchlässig bei mindestens einer ausgewählten Wellenlänge sind.

4. Säule mit mehreren Lagen nach Anspruch 1, bei der eine jede der Vielzahl der Membranlagen eine Einfanglage, die ein Antianalyt trägt, und mindestens eine lichtabschirmende Lage im wesentlichen koplanar mit der Einfanglage aufweist.

5. Säule mit mehreren .Lagen nach Anspruch 4, bei der mindestens eine der lichtabschirmenden Lagen eine Lichtabsorptionsunterlage umfaßt.

6. Säule mit mehreren Lagen nach Anspruch 4, bei der mindestens eine der lichtabschirmenden Lagen eine Lichtreflexionsunterlage umfaßt.

7. Säule mit mehreren Lagen nach Anspruch 1, bei der mindestens eine Vielzahl von Trägermaterialien in fester Phase eine Blockierungssubstanz trägt.

8. Säule mit mehreren Lagen nach Anspruch 1, bei der im wesentlichen alle Flächen innerhalb der Kammer eine Blockierungssubstanz tragen.

9. Säule mit mehreren Lagen nach Anspruch 1, bei der die hintersten Membranlagen der Vielzahl von Membranlagen Filterlagen mit Poren sind, die so bemessen sind, daß sie im wesentlichen den Fluß von Teilchen mit einer vorgewählten Größe zu den mittleren Membranlagen der Vielzahl von Membranlagen verhindern.

10. Säule mit mehreren Lagen nach Anspruch 1, bei der die Membranlagen axial voneinander längs der Längsachse beabstandet sind.

11. Säule mit mehreren Lagen nach Anspruch 1, die außerdem eine Pufferlösung aufweist, die innerhalb der Kammer enthalten ist.

12. Säule mit mehreren Lagen nach Anspruch 1, bei der die Kammer eine zweite Öffnung an einem zweiten Ende davon aufweist.

13. Säule mit mehreren Lagen nach Anspruch 12, die außerdem einen Abproduktbehälter in Fluidverbindung mit der zweiten Öffnung der Kammer aufweist.

14. Säule mit mehreren Lagen nach Anspruch 1, die außerdem eine Fluidflußöffnung aufweist, um einen Fluidaustrittsflußweg aus der Kammer heraus bereitzustellen.

15. Säule mit mehreren Lagen nach Anspruch 1, bei der ein jeder der Antianalyte aus der Gruppe ausgewählt wird, die besteht aus: Antikörpern; Antigenen; Liganden; Ligandenrezeptoren; Nukleinsäuren, die an einer Analyt-Nukleinsäure hybridisieren können; enzymverbundenes Immunosorbens-Analyt; Proteine oder Bruchstücke von Proteinen, die in der Lage sind, einen Komplex mit einem Analyt-Protein oder Protein-Bruchstück zu bilden; und chemische Verbindungen, die in der Lage sind, eine biologische Aktivität mit einem Target-Analyt zu zeigen.

16. Fluidtestvorrichtung, die in Kombination aufweist:
ein Gehäuse;
eine Montageeinrichtung innerhalb des Gehäuses, um eine längliche Kammer aufzunehmen;
eine längliche Kammer, die in der Montageeinrichtung montiert ist, die eine erste Öffnung in einem ersten Ende davon und eine Vielzahl von vertikal gestapelten, im wesentlichen ebenen filterartigen mikroporösen Membranlagen aufweist, die innerhalb der Kammer gestapelt sind, die mindestens eine Vielzahl von Trägermaterialien in fester Phase umfaßen, wobei jedes ein unterschiedliches Antianalyt trägt, und wobei jede Membranlage eine Vielzahl von Poren aufweist, die den Fluß eines Probefluids durch jede Membranlage gestatten, und was gestattet, daß das Probefluid durch die Vielzahl der vertikal gestapelten Membranlagen fließt, wobei die Ebene einer jeden der Membanlagen im wesentlichen senkrecht zur Längsachse der Kammer verläuft;
einen Reagensbehälter und eine Fluidliefereinrichtung innerhalb des Gehäuses, um ein Reagens aus dem Reagensbehälter zur ersten Öffnung der länglichen Kammer zuzuführen;
einen Waschpufferbehälter und eine zweite Fluidliefereinrichtung innerhalb des Gehäuses, um den Waschpuffer aus dem Waschpufferbehälter zur ersten Öffnung der länglichen Kammer zuzuführen; und
einen Sensor innerhalb des Gehäuses, um ein Signal von der länglichen Kammer zu empfangen, und um ein entsprechendes elektrisches Signal zu erzeugen.

17. Fluidtestvorrichtung nach Anspruch 16, die außerdem einen Computer aufweist, der auf das elektrische Signal anspricht, das vom Sensor erzeugt wird.

18. Fluidtestvorrichtung nach Anspruch 16, die außerdem ein Fluidtransportsystem aufweist, das eine Fluidprobenbehältermontageeinrichtung, um mindestens einen Behälter mit der Fluidprobe zu montieren, und eine Fluidprobenliefereinrichtung aufweist, um die Fluidprobe vom Behälter mit der Fluidprobe zu einer länglichen Kammer zuzuführen, die in der Montageeinrichtung montiert ist.

19. Fluidtestvorrichtung nach Anspruch 18, bei der die Fluidprobenbehältermontageeinrichtung gleichzeitig eine Vielzahl von Behältern mit der Fluidprobe hält, und bei der die Fluidprobenliefereinrichtung so angepaßt ist, daß sie aufeinanderfolgend die Fluidprobe von jedem der Behälter mit der Fluidprobe zu einer entsprechenden länglichen Kammer zuführt.

20. Fluidtestvorrichtung nach Anspruch 16, die außerdem eine Erregungsenergiequelle innerhalb des Gehäuses aufweist, um Erregungsenergie in Richtung der länglichen Kammer zu lenken.

21. Fluidtestvorrichtung nach Anspruch 20, bei der die Erregungsenergiequelle eine Lichtquelle aufweist, die ausgerichtet ist, um mindestens eine der Membranlagen in der länglichen Säule mit Licht von einer vorgewählten Wellenlänge zu beleuchten, worin mindestens eine der Membranlagen in der länglichen Kammer auf die vorgewählte Wellenlänge anspricht.

22. Fluidtestvorrichtung nach Anspruch 20, bei der die Erregungsenergiequelle ein Spannungsdifferential über die längliche Kammer anlegt.

23. Fluidtestvorrichtung nach Anspruch 16, die außerdem eine regelbare Druckquelle aufweist, die funktionell mit der ersten Öffnung verbunden ist.

## Revendications

1. Colonne multicouche comprenant, en combinaison:
une chambre qui comporte un axe longitudinal avec une première extrémité comportant une première ouverture; et
une pluralité de couches de membrane microporeuse similaires à un filtre sensiblement planes, empilées verticalement, qui sont empilées dans la chambre, incluant au moins une pluralité de substrats en phase solide dont chacun supporte un anti-analyte différent et chaque couche de membrane comportant une pluralité de pores, ce qui autorise un fluide échantillon à circuler au travers de chaque couche de membrane et ce qui permet au fluide échantillon de circuler au travers de la pluralité de couches de membrane empilées verticalement, le plan de chacune des couches de membrane étant sensiblement perpendiculaire à l'axe longitudinal de la chambre.

2. Colonne multicouche selon la revendication 1, dans laquelle chacune des couches de membrane comprend une membrane à matrice de fibres.

3. Colonne multicouche selon la revendication 1, dans laquelle les couches de membrane sont sensiblement transparentes à une lumière d'au moins une longueur d'onde sélectionnée.

4. Colonne multicouche selon la revendication 1, dans laquelle chacune de la pluralité de couches de membrane comprend une couche de capture qui supporte un anti-analyte et au moins une couche de protection vis-à-vis de la lumière sensiblement coplanaire à la couche de capture.

5. Colonne multicouche selon la revendication 4, dans laquelle au moins l'une des couches de protection vis-à-vis de la lumière inclut une sous-couche d'absorption de lumière.

6. Colonne multicouche selon la revendication 4, dans laquelle au moins l'une des couches de protection vis-à-vis de la lumière inclut une sous-couche de réflexion de lumière.

7. Colonne multicouche selon la revendication 1, dans laquelle au moins une pluralité de substrats en phase solide supportent une substance de blocage.

8. Colonne multicouche selon la revendication 1, dans laquelle sensiblement toutes les surfaces dans la chambre supportent une substance de blocage.

9. Colonne multicouche selon la revendication 1, dans laquelle des couches de membrane situées le plus en extrémité de la pluralité de couches de membrane sont des couches de filtre présentant des pores dimensionnés de manière à empêcher de façon substantielle la circulation de particules d'une dimension présélectionnée jusqu'à des couches de membrane centrales de la pluralité de couches de membrane.

10. Colonne multicouche selon la revendication 1, dans laquelle les couches de membrane sont espacées axialement les unes des autres le long de l'axe longitudinal.

11. Colonne multicouche selon la revendication 1, comprenant en outre une solution tampon qui est contenue à l'intérieur de la chambre.

12. Colonne multicouche selon la revendication 1, dans laquelle la chambre comporte une seconde ouverture au niveau d'une seconde extrémité d'elle-même.

13. Colonne multicouche selon la revendication 12, comprenant en outre un réservoir de rejet en communication de fluide avec la seconde ouverture de la chambre.

14. Colonne multicouche selon la revendication 1, comprenant en outre un orifice de circulation de fluide pour assurer une voie de circulation de sortie de fluide depuis l'intérieur de la chambre.

15. Colonne multicouche selon la revendication 1, dans laquelle chacun des anti-analytes est choisi parmi le groupe comprenant des anticorps, des antigènes, un ligand, des récepteurs de ligand, des acides nucléiques susceptibles d'une hybridation vis-à-vis d'un acide nucléique d'analyte, un analyte immuno-sorbant lié par enzyme, des protéines ou des fragments de protéine susceptibles de former un complexe avec une protéine ou un fragment de protéine d'analyte et des composés chimiques susceptibles de présenter une activité biologique avec l'analyte cible.

16. Appareil de test de fluide comprenant, en combinaison:
un boîtier;
un moyen de montage à l'intérieur du boîtier pour recevoir une chambre allongée;
une chambre allongée qui est montée dans le moyen de montage, qui comporte une première ouverture au niveau d'une première extrémité d'elle-même et une pluralité de couches de membrane microporeuse similaires à un filtre sensiblement planes, empilées verticalement, qui sont empilées dans la chambre, incluant au moins une pluralité de substrats en phase solide dont chacun supporte un anti-analyte différent et chaque couche de membrane comportant une pluralité de pores, ce qui autorise un fluide échantillon à circuler au travers de chaque couche de membrane et ce qui permet au fluide échantillon de circuler au travers de la pluralité de couches de membrane empilées verticalement, le plan de chacune des couches de membrane étant sensiblement perpendiculaire à l'axe longitudinal de la chambre;
un réservoir de réactif et un moyen de délivrance de fluide à l'intérieur du boîtier pour alimenter un réactif depuis le réservoir de réactif jusqu'à la première ouverture de la chambre allongée;
un réservoir de tampon de lavage et un second moyen de délivrance de fluide à l'intérieur du boîtier pour alimenter un tampon de lavage depuis le réservoir de tampon de lavage jusqu'à la première ouverture de la chambre allongée; et
un capteur à l'intérieur du boîtier pour recevoir un signal depuis la chambre allongée et pour générer un signal électrique correspondant.

17. Appareil de test de fluide selon la revendication 16, comprenant en outre un ordinateur sensible au signal électrique qui est généré par le capteur.

18. Appareil de test de fluide selon la revendication 16, comprenant en outre un système de manipulation de fluide qui comprend un moyen de montage de conteneur d'échantillon de fluide pour monter au moins un conteneur d'échantillon de fluide et un moyen de délivrance d'échantillon de fluide pour alimenter un échantillon de fluide depuis le conteneur d'échantillon de fluide jusqu'à une chambre allongée qui est montée dans le moyen de montage.

19. Appareil de test de fluide selon la revendication 18, dans lequel le moyen de montage de conteneur d'échantillon de fluide contient de façon simultanée une pluralité de conteneurs d'échantillon de fluide et le moyen de délivrance d'échantillon de fluide est adapté pour alimenter de façon séquentielle l'échantillon de fluide depuis chacun des conteneurs d'échantillon de fluide jusqu'à une chambre allongée correspondante.

20. Appareil de test de fluide selon la revendication 16, comprenant en outre une source d'énergie d'excitation à l'intérieur du boîtier pour diriger une énergie d'excitation en direction de la chambre allongée.

21. Appareil de test de fluide selon la revendication 20, dans lequel la source d'énergie d'excitation comprend une source de lumière orientée pour éclairer au moins l'une des couches de membrane dans la colonne allongée à l'aide d'une lumière d'une longueur d'onde présélectionnée, dans lequel au moins une des couches de membrane dans la chambre allongée est sensible à la longueur d'onde présélectionnée.

22. Appareil de test de fluide selon la revendication 20, dans lequel la source d'énergie d'excitation applique une différence de tension sur la chambre allongée.

23. Appareil de test de fluide selon la revendication 16, comprenant en outre une source de pression variable connectée en fonctionnement à la première ouverture.
